# FASCICULE DE BREVET EUROPEEN

(11) **EP 1 097 703 B1**
(45) Date de publication et mention de la délivrance du brevet: **09.01.2002**
(21) Numéro de dépôt: 00402648.0
(22) Date de dépôt: 25.09.2000
(51) Int. Cl.: A61K 7/48, A61K 7/06

(54) **Composition à application topique contenant un sucre, et ses utilisations cosmétiques**
Topische Zusammensetzung enthaltend einen Zucker und ihre Verwendung in der Kosmetik
Topical composition containing a sugar and its use in cosmetics

(30) Priorité: 08.11.1999 FR 9913990
(43) Date de publication de la demande: 09.05.2001
(73) Titulaire: L'OREAL, 75008 Paris (FR)
(72) Inventeur: Lorant, Raluca, 94320 Thiais (FR)
(74) Mandataire: Rasson, Catherine

(56) Documents cités:
- EP-A- 0 545 002
- WO-A-97/32561
- US-A- 5 266 321

## Description

La présente invention se rapporte à une composition contenant un sucre et/ou un dérivé de sucre et une suspension aqueuse de particules d'organopolysiloxane élastomère au moins partiellement réticulé. L'invention se rapporte aussi aux utilisations notamment cosmétiques de cette composition, en particulier pour le soin, . le nettoyage et/ou le démaquillage de la peau, des muqueuses, des yeux et/ou des cheveux. L'invention se rapporte aussi à l'utilisation d'une suspension aqueuse de particules d'un organopolysiloxane, dans une composition contenant au moins un sucre et/ou un dérivé de sucre, afin d'éliminer le toucher collant dû à ce sucre ou dérivé de sucre.

Pour leur apporter un meilleur confort d'utilisation (douceur, émollience et autres), il est courant d'introduire dans les compositions cosmétiques actuelles, des sucres ou des dérivés de sucre, du fait que ces composés apportent notamment de la douceur et une meilleure tolérance des produits. Ainsi, les tensioactifs dérivés de sucre sont plus doux et moins irritants que les tensioactifs classiques. Par ailleurs, les sucres ont de bonnes propriétés hydratantes et sont donc incorporés dans les compositions cosmétiques comme hydratants.

Toutefois, les sucres et leurs dérivés présentent l'inconvénient de donner un caractère collant aux compositions les contenant.

Il subsiste donc le besoin d'une composition à application topique, notamment cosmétique, contenant un sucre et/ou un dérivé de sucre, et ne conférant pas de sensation de collant sur la peau.

De façon surprenante, la demanderesse a trouvé qu'il était possible de supprimer le toucher collant sur la peau, d'une composition contenant un sucre et/ou un dérivé de sucre, en introduisant une suspension aqueuse de particules d'un organopolysiloxane élastomère au moins partiellement réticulé.

Certes, les documents EP-A-545002, WO-A-97/32561 et US-A-5,266,321 décrivent des compositions cosmétiques contenant des particules d'organopolysiloxane élastomère et pouvant contenir aussi des esters de sucre. Toutefois, les particules d'organopolysiloxane élastomère décrites dans ces documents sont sous forme de gels huileux et notamment siliconés dans lesquels les particules gonflent, tandis que les particules utilisées selon la présente invention sont dispersées dans un milieu aqueux dans lesquelles elles ne gonflent pas. Les gels huileux décrits dans les documents cités ci-dessus sont introduits dans une phase huileuse et non dans une phase aqueuse, et de ce fait ils n'apportent pas de sensation de fraîcheur lors de l'application sur la peau. En outre, ils n'apportent pas de suppression de l'effet collant des sucres ou dérivés de sucre, contrairement aux particules en suspension aqueuse utilisées dans la présente invention.

Aussi, l'invention se rapporte à une composition contenant dans un milieu physiologiquement acceptable, au moins une phase aqueuse, au moins un sucre et/ou au moins un dérivé de sucre et une suspension aqueuse de particules d'organopolysiloxane élastomère au moins partiellement réticulé, ladite suspension étant présente dans la phase aqueuse de la composition.

On entend par "physiologiquement acceptable" un milieu compatible avec la peau, les yeux et les fibres kératiniques des êtres humains.

Par ailleurs, on entend dans la présente demande par « sucre » des composés qui possèdent plusieurs fois la fonction alcool avec ou sans fonction aldéhyde ou cétone et qui comportent au moins 4 atomes de carbone, et de préférence de 5 à 6 atomes de carbone.

Du fait que les particules d'organopolysiloxane élastomère au moins partiellement réticulé sont en suspension aqueuse et donc introduites dans une phase aqueuse, la composition obtenue est beaucoup plus fraîche et agréable à l'application que si ces particules étaient dans la phase huileuse. Cet effet de fraîcheur est particulièrement appréciable dans le cas des compositions comportant une phase huileuse, et notamment dans le cas des émulsions huile-dans-eau (H/E) ou eau-dans-huile (E/H).

Par ailleurs, comme indiqué ci-dessus, la présence de la suspension aqueuse de particules d'organopolysiloxane élastomère dans la composition permet d'éviter l'effet collant apporté par le sucre ou le dérivé de sucre.

Aussi, la présente invention se rapporte encore à l'utilisation d'une suspension aqueuse de particules d'organopolysiloxane élastomère au moins partiellement réticulé, dans une composition contenant au moins un sucre et/ou un dérivé de sucre, afin d'éliminer le toucher collant dû à ce sucre et/ou dérivé de sucre.

Par « élastomère » on entend un matériau souple, déformable ayant des propriétés viscoélastiques et notamment la consistance d'une éponge ou d'une sphère souple. Son modèle d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomères de la composition de l'invention ne sont pas desséchants pour la peau et apportent de bonnes propriétés cosmétiques, notamment de douceur et de matité. Ces élastomères conduisent à des compositions confortables à l'application, de bon étalement, douces, non collantes au toucher et non grasses.

Les organopolysiloxanes élastomères conformes à l'invention sont partiellement ou totalement réticulés et de structure tridimensionnelle.

Les élastomères de l'invention se présentent sous forme de gel émulsionné contenant un organopolysiloxane élastomère de structure tridimensionnelle, dispersé dans de l'eau. La dispersion (ou suspension) des particules est homogène. Ces élastomères peuvent être choisis notamment parmi les polymères réticulés décrits dans la demande JP-A-10/175816. Selon cette demande, ils sont obtenus par réaction d'addition et de réticulation, en présence d'un catalyseur en particulier du type platine, d'au moins :
- (a) un organopolysiloxane (i) ayant au moins deux groupes vinyliques en position α-ω de la chaîne siliconée par molécule ; et
- (b) un organosiloxane (ii) ayant au moins un atome d'hydrogène lié à un atome de silicium par molécule.

En particulier, l'organopolysiloxane (i) est choisi parmi les polydiméthylsiloxanes et est plus spécifiquement un α-ω-diméthylvinyl polydiméthylsiloxane.

La suspension aqueuse de particules d'organopolysiloxane élastomère, utilisée dans la composition selon l'invention peut être notamment obtenue comme suit :
- (a) mélange de l'organopolysiloxane (i) et de l'organosiloxane (ii) ;
- (b) ajout de la phase aqueuse contenant un émulsifiant au mélange de l'étape (a);
- (c) émulsification de la phase aqueuse et dudit mélange ;
- (d) ajout d'eau chaude à l'émulsion de l'étape (c) ; et
- (e) polymérisation de l'organopolysiloxane (i) et de l'organosiloxane (ii) en émulsion en présence d'un catalyseur de platine.

De préférence, l'étape (c) est obtenue en présence d'un émulsifiant non-ionique.

Dans ce procédé, l'eau est avantageusement ajoutée à une température d'environ 40 à 60°C. Après l'étape (e), il est possible de sécher les particules obtenues, pour en évaporer toute ou une partie de l'eau piégée.

Les organopolysiloxanes sont sous la forme de particules solides déformables ayant une certaine dureté, mesurable avec un duromètre Shore A (selon la norme ASTM D2240) à la température ambiante ou avec la méthode japonaise JIS-A. Cette dureté peut être mesurée sur un bloc d'élastomère préparé à cet effet comme suit : mélange de l'organopolysiloxane (i) et de l'organosiloxane (ii) ; élimination de l'air du mélange; moulage et vulcanisation au four à 100°C pendant 30 minutes ; refroidissement à température ambiante puis mesure de la dureté. La densité est aussi déterminée sur ce bloc d'élastomère.

En particulier, la dureté Shore est inférieure ou égale à 80 et mieux inférieure à 65. Les organopolysiloxanes de la composition de l'invention sont par exemple ceux commercialisés sous les noms BY 29-122 et BY 29-119 par la société Dow-Corning Electric. On peut aussi utiliser un mélange de ces produits commerciaux. Un bloc d'élastomères selon le produit BY 29-122 présente une dureté de 7 et selon le produit BY 29-119 une dureté de 30. La densité est de 0,97 à 0,98. Les suspensions de noms BY 29-122 et BY 29-119 comportent environ 63 % en poids de particules d'organopolysiloxane élastomère (donc environ 63 % de matière active) par rapport au poids total de la suspension.

En particulier, les particules d'organopolysiloxane élastomère (en matière active) ont une taille allant de 0,1 à 500 µm et mieux de 3 à 200 µm. Ces particules peuvent être sphériques, plates ou amorphes avec, de préférence, une forme sphérique.

Ainsi qu'indiqué dans le procédé décrit ci-dessus, ces particules d'organopolysiloxane, pour se disperser de façon stable dans l'eau, peuvent être associées à un ou plusieurs émulsifiants non ioniques, cationiques ou anioniques de HLB (Hydrophilic lipophilic balance) supérieur ou égal à 8. La proportion d'émulsifiants dans la suspension aqueuse va de préférence de 0,1 à 20 parties en poids pour 100 parties en poids de la suspension aqueuse d'organopolysiloxane élastomère, et mieux, de 0,5 à 10 parties en poids (voir description du document JP-A-10/175816).

En outre, à ces particules d'organopolysiloxane élastomère, peuvent être associées dans la suspension aqueuse, des corps gras, et notamment des huiles telles que celles décrites dans le document JP-A-10 /175816, des cires ou des gommes solides à température ambiante, des corps gras pâteux, d'origine animale, végétale, minérale ou synthétique, leurs mélanges ainsi que des poudres inorganiques telles que celles décrites dans ce document.

De façon préférentielle, les particules d'organopolysiloxane élastomère sont présentes dans la composition de l'invention en une quantité en matière active allant par exemple de 0,1 à 30 % en poids et de préférence de 0,5 à 20 % en poids par rapport au poids total de la composition.

Comme sucres utilisables dans la composition de l'invention, on peut citer par exemple le sucrose (ou saccharose), le glucose, le galactose, le ribose, le fucose, le tréhalose, le maltose, le fructose, le mannose, l'arabinose, le xylose, le lactose, leurs dérivés notamment alkylés, tels que les dérivés méthylés comme le méthylglucose, ainsi que les composés contenant un ou plusieurs sucres, et leurs mélanges. Comme composé contenant un sucre ou un mélange de sucres, on peut citer les composés naturels comme le miel, et les polymères comme par exemple le produit commercialisé sous la dénomination « fucogel 1000 » par la société Solabia (nom CTFA Biosaccharide gum-1), polymère contenant du fucose, du galactose et de l'acide galacturonique.

Comme dérivés de sucre utilisables dans la composition de l'invention, on peut citer notamment les esters gras de sucre éventuellement oxyalkylénés (oxyéthylénés et/ou oxypropylénés) ou polyglycérolés, et les éthers gras de sucre. Ces composés peuvent être en particulier utilisés comme tensioactifs émulsionnants.

Les esters d'acide gras et de sucre, utilisables dans la composition selon l'invention peuvent être choisis notamment dans le groupe comprenant les esters ou les mélanges d'esters d'acides gras en C₁₂ à C₂₂ linéaires ou ramifiés, saturés ou insaturés et de sucrose, de maltose, de glucose, de fructose, de mannose, de galactose, d'arabinose, de xylose, de lactose, de tréhalose, ou de méthylglucose.

De préférence ces esters seront choisis parmi les mono-, di-, tri- et tétraesters, les polyesters et leurs mélanges. Ces esters peuvent être notamment choisis parmi les stéarates, béhénates, cocoates, arachidonates, palmitates, myristates, laurates, caprates, oléates, laurates, et leurs mélanges.

On utilise de préférence les esters de sucrose. On peut citer par exemple comme ester de sucrose, le cocoate de sucrose, le monooctanoate de sucrose, le monodécanoate de sucrose, le mono- ou di-laurate de sucrose, le monomyristate de sucrose, le mono- ou di-palmitate de sucrose, le mono- et di-stéarate de sucrose, le mono-, di-, tri-oléate de sucrose, le mono- ou di-linoléate de sucrose, les polyesters de sucrose comme le pentaoléate, l'hexaoléate, l'heptaoléate, l'octooléate de sucrose, et les esters mixtes comme le palmito-stéarate de sucrose.

On peut citer, à titre d'exemples d'esters ou de mélanges d'esters d'acide gras et de sucrose, ceux vendus par la société CRODESTA sous les dénominations F160, F140, F110, F90, F70, SL40, désignant respectivement les palmitostéarates de sucrose formés de 73 % monoester et 27 % di- et tri-ester, de 61 % monoester et 39 % di-tri-tétraester, de 52 % monoester et 48 % di-tri-tétraester, de 45 % monoester et 55 % di-, tri- et tétra-ester, de 39 % monoester et 61 % de di-, tri-, et tétra-ester, et le monolaurate de sucrose. On peut aussi utiliser ceux vendus par la société MITSUBISHI sous la dénomination Ryoto Sugar esters par exemple sous la référence B370 et correspondant au béhénate de saccharose formé de 20 % monoester et 80 % di-triester-polyester. On peut aussi citer le mono-di-palmitostéarate de sucrose commercialisé par la société Goldschmidt sous la dénomination « Tegosoft PSE ». On peut aussi utiliser un mélange de ces différents produits.

L'ester de sucre peut aussi être en mélange avec un autre composé non dérivé de sucre, et ainsi on peut citer par exemple le mélange de stéarate de sorbitan et de cocoate de sucrose, commercialisé sous la dénomination « Arlatone 2121 » par la société ICI.

Comme autres esters de sucre, on peut citer par exemple le trioléate de glucose, le di-, tri-, tétra- ou penta-oléate de galactose, le di-, tri- ou tétra- linoléate d'arabinose, le di-, tri- ou tétra-linoléate de xylose.

A titre d'exemples d'esters ou de mélanges d'esters d'acide gras et de méthylglucose, on peut citer le distéarate de méthyl glucose et de polyglycérol-3, vendu par la société Goldschmidt sous la dénomination de Tegocare 450. On peut citer aussi les monoesters de glucose ou de maltose tels que l'O-hexadécanoyle-6-D-glucoside de méthyle et l'O-hexadécanoyle-6-D-maltose.

A titre d'exemples d'esters d'acide gras et de sucre oxyéthylénés, on peut citer le sesquistéarate de méthylglucose oxyéthyléné (20 OE) tel que le produit commercialisé sous la dénomination « Glucamate SSE-20 » par la société Amerchol.

Les éthers d'alcool gras et de sucre, utilisables comme tensioactifs dans la composition selon l'invention peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.

Les alcools gras en C₈-C₂₂ ou en C₁₄-C₂₂ formant le motif gras des éthers de sucre comportent une chaîne alkyle linéaire saturée ou non saturée, comportant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanyle, et leurs mélanges tels que cétéaryle.

A titre d'exemples d'éthers d'alcool gras et de sucre, on peut citer les alkylpolyglucosides tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. On peut citer aussi les alkylpolyglycosides de HLB (Balance hydrophile lipophile) inférieur à 7, utilisables notamment dans les émulsions E/H, tels que l'isostéaryl-glucoside éventuellement en mélange avec l'alcool isostéarylique, commercialisé par exemple sous la dénomination Montanov WO18 par la société Seppic, et l'oléyl-glucoside éventuellement en mélange avec l'alcool oléylique, commercialisé par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

Les sucres ou dérivés de sucre peuvent être introduits dans la phase aqueuse ou une phase huileuse de la composition de l'invention. De manière générale, les sucres sont introduits dans la phase aqueuse. Quant aux dérivés de sucre, ils sont introduits dans la phase aqueuse ou une phase huileuse selon leurs caractères hydrophiles ou lipophiles.

La quantité de sucre(s) et/ou dérivé(s) de sucre dans la composition de l'invention peut varier dans une large mesure selon le but recherché. Elle peut aller par exemple de 0,1 à 20 % en poids et de préférence de 0,5 à 10 % en poids par rapport au poids total de la composition.

La composition selon l'invention est appropriée notamment pour une utilisation topique et elle peut constituer en particulier une composition cosmétique et/ou dermatologique.

Cette composition peut se présenter sous toutes les formes galéniques normalement utilisées dans les domaines cosmétique et dermatologique, et elle peut être notamment sous forme d'une solution aqueuse éventuellement gélifiée, d'un gel aqueux, d'une dispersion, d'une émulsion obtenue par dispersion d'une phase huileuse dans une phase aqueuse (H/E) ou inversement (E/H), ou d'une émulsion triple (E/H/E ou H/E/H). Elles peuvent également se trouver sous une forme vectorisée, comme par exemple sous forme de nanocapsules, de liposomes, de nanoémulsions, d'oléosomes. Ces compositions sont préparées selon les méthodes usuelles.

Selon un mode préféré de réalisation de l'invention, la composition est sous forme d'une émulsion H/E ou E/H, et préférentiellement H/E.

Lorsque la composition est une émulsion, cette dernière contient de façon classique au moins une huile et éventuellement un émulsionnant approprié qui peut être notamment un dérivé de sucre.

La nature de la phase huileuse rentrant dans la composition des émulsions n'est pas critique et elle peut ainsi être constituée par tous les corps gras et notamment les huiles, classiquement utilisés dans les domaines cosmétique et dermatologique.

Parmi les huiles utilisables dans l'émulsion de l'invention, on peut notamment citer par exemple les huiles végétales (par exemple de noyaux d'abricot, jojoba, avocat, sésame, tournesol, maïs, soja, carthame, pépins de raisin, fraction liquide de beurre de karité, perhydrosqualène végétal), les huiles minérales (vaseline, isoparaffines telles que isohexadécane, di-octylcyclohexane), les huiles de synthèse (caprylic/capric triglycérides, myristate d'isopropyle, octanoate de cétéaryle, polyisobutylène, palmitate d'éthyl-hexyle, alkylbenzoates), les huiles de silicone volatiles (cyclométhicone telle que le cyclohexaméthicone) ou non volatiles, et les huiles fluorées. Les autres corps gras susceptibles d'être présents dans la phase huileuse peuvent être par exemple les acides gras tels que l'acide stéarique, les alcools gras tels que l'alcool stéarylique, et les cires.

La phase huileuse de l'émulsion peut représenter par exemple de 1 à 50 % et mieux de 5 à 40 % en poids du poids total de l'émulsion.

Les émulsions peuvent contenir au moins un agent émulsionnant permettant de stabiliser l'interface huile/eau. L'agent émulsionnant peut être choisi parmi les agents émulsionnants dérivés de sucre, mais peut être constitué aussi de tensioactifs amphotères, anioniques, cationiques ou non-ioniques, utilisés seuls ou en mélange. Ces agents émulsionnants sont choisis de manière appropriée suivant l'émulsion à obtenir (E/H ou H/E).

En outre, de façon connue, les compositions de l'invention peuvent contenir des adjuvants habituels dans les domaines cosmétique et dermatologique, tels que les actifs hydrophiles ou lipophiles, les conservateurs, les antioxydants, les parfums, les charges, les matières colorantes (pigments ou colorants), les filtres solaires, et encore les vésicules lipidiques. Ces adjuvants sont utilisés dans les proportions habituelles dans le domaine cosmétique ou dermatologique, et par exemple de 0,01 à 20 % du poids total de l'émulsion, et ils sont, selon leur nature, introduits dans la phase aqueuse ou dans la phase huileuse de la composition, ou encore dans des vésicules. Ils sont, selon leur nature, introduits dans l'une ou l'autre phase de la composition, ou encore dans des vésicules.

Bien entendu, l'homme du métier veillera à choisir ce ou ces éventuels composés complémentaires et/ou leur quantité de manière telle que les propriétés avantageuses de la composition selon l'invention ne soient pas, ou substantiellement pas, altérées par l'adjonction envisagée.

Comme actifs, on peut citer notamment les filtres solaires ; les vitamines et notamment les vitamines A (rétinol), C (acide ascorbique), E (tocophérol), B3 (niacinamide), F et D et leurs dérivés ; les acides gras insaturés comme l'acide linoléique et l'acide linolénique ; l'alpha-bisabolol ; les beurres d'origine végétale citées ci-dessus parmi les huiles, comme le beurre de Shorea ou le beurre de karité qui reconstituent la barrière lipidique de la peau et permettent le traitement des peaux sèches ; l'urée ; la rutine ; les enzymes ; les extraits naturels tels que le thé vert, l'extrait de mélisse, l'extrait de thym, les oligomères procyannidoliques (OPC) tels que l'OPC d'aubépine, l'OPC de pin et l'OPC de raisin ; certains acides tels que l'acide kojique, l'acide caféique, l'acide rétinoique et ses dérivés, l'acide benzène 1,4-di-(3-méthylidène 10-camphosulfonique) ; les caroténoïdes tels que les carotènes comme par exemple les α-, β- et γ-carotènes, le β,ϕ-carotène, le ξ-carotène, le β,λ-carotène, le lycopène (ψ,ψ-carotène), et leurs mélanges.

Selon la fluidité de la composition que l'on souhaite obtenir, on peut y ajouter un ou plusieurs gélifiants comme les argiles, les gommes polysaccharides et leurs dérivés (gomme de xanthane) les polymères carboxyvinyliques ou carbomers, les polyacrylamides et les copolymères d'acrylamide, tels que le produit vendu sous le nom de SEPIGEL 305 par la société Seppic, et le produit vendu sous le nom de HOSTACERIN AMPS par la société Hoechst (nom CTFA : Ammonium polyacryldimethyltauramide). Ces gélifiants sont généralement utilisés à des concentrations allant de 0,1 à 10 %, de préférence de 0,1 à 5 % et mieux de 0,1 à 3 % du poids total de la composition.

Comme charges susceptibles d'être utilisées dans la composition de l'invention, on peut citer par exemple les particules de polyamide et notamment celles vendues sous la dénomination Orgasol par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de Polytrap ; les poudres expansées telles que les microsphères creuses et notamment, les microsphères commercialisées sous la dénomination commerciale Expancel par la société Kemanord Plast ou sous la dénomination commerciale Micropearl F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination Dry-Flo par la société National Starch ; les microbilles de résine de silicone telles que celles commercialisées sous la dénomination Tospearl par la société Toshiba Silicone ; et leurs mélanges.

Les compositions, objets de l'invention, trouvent notamment leur application dans un grand nombre de traitements cosmétiques de la peau, des lèvres et des cheveux, y compris le cuir chevelu, notamment pour le traitement, la protection, le soin, le démaquillage et/ou le nettoyage de la peau, des lèvres et/ou des cheveux. Elles peuvent être destinées aussi au traitement des peaux sèches et/ou des lèvres sèches et/ou des peaux sensibles.

Les compositions selon l'invention peuvent par exemple être utilisées comme produits de soin, de démaquillage et/ou de nettoyage pour le visage sous forme de crèmes ou de laits.

Aussi, l'invention a encore pour objet l'utilisation cosmétique de la composition telle que définie ci-dessus pour le traitement, la protection, le soin, le démaquillage et/ou le nettoyage de la peau, des lèvres et/ou des cheveux.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une composition telle que définie ci-dessus.

L'invention a aussi pour objet l'utilisation de la composition telle que définie ci-dessus pour la fabrication d'une composition destinée au soin des peaux sèches et/ou des lèvres sèches et/ou des peaux sensibles.

Les exemples de compositions données ci-après sont donnés à titre illustratif et non limitatif. Les quantités sont données en pourcentage en poids, sauf mention contraire.

### Exemple 1 : Emulsion huile-dans-eau

| *Phase aqueuse* | |
|---|---|
| Glycérol | 5 % |
| BY 29-122 (à 63 % de matière active) | 5 % |
| Conservateurs | 0,3 % |
| Miel | 0,5 % |
| Eau | qsp 100 % |

| *Phase huileuse* | |
|---|---|
| Palmito-stéarate de sucrose (Tegosoft PSE de la société Goldschmidt) | 3 % |
| Acide stéarique | 1 % |
| Alcool stéarylique | 3 % |
| Huile de vaseline | 10 % |
| Perhydrosqualène végétal | 10 % |
| Cyclohexaméthicone | 5 % |

| *Gélifiant* | |
|---|---|
| Sepigel 305 | 1 % |

Mode opératoire : On chauffe séparément les deux phases à 70°C puis on disperse la phase huileuse dans la phase aqueuse sous agitation. Ensuite, à une température d'environ 60°C, on ajoute le gélifiant.

On obtient une crème onctueuse, très riche, douce et apaisante. Malgré le fort taux de corps gras cette crème n'est ni collante ni grasse.

### Exemple 2 : Emulsion eau-dans-huile

| *Phase huileuse :* | |
|---|---|
| Cétyl dimethicone copolyol (émulsionnant) | 1,5 % |
| Polyglycéryl-4 isostéarate (co-émulsionnant) | 0,5 % |
| Isohexadécane | 5 % |
| Cyclohexamethicone | 10 % |
| Perhydrosqualène | 10,5 % |

| *Charges :* | |
|---|---|
| Expancel 551 | 0,5 % |
| Amidon réticulé (Dry Flo) | 2 % |

| *Phase aqueuse:* | |
|---|---|
| Glycérine | 5 % |
| Sulfate de magnésium | 0,5 % |
| Fucogel 1000 (de la société Solabia) | 3 % |
| Conservateurs | 0,4 % |
| BY 29-122 (à 63 % de matière active) | 5 % |
| Eau | qsp 100 % |

Mode opératoire : on disperse les charges dans la phase huileuse, puis on disperse très doucement sous agitation vigoureuse la phase aqueuse dans le mélange obtenu.

On obtient une crème de soin appropriée pour le soin des peaux sèches.

### Exemple 3 : Emulsion eau-dans-huile

| *Phase huileuse :* | |
|---|---|
| Isostéaryl-glucoside/alcool isostéarylique (15/85) (soit 0,45 % de matière active d'alkyl-polyglucoside) | 3 % |
| Huile de noyaux d'abricot | 8 % |
| Cyclohexaméthicone | 8 % |
| Cire | 2 % |

| *Phase aqueuse :* | |
|---|---|
| Glycérine | 5 % |
| Sulfate de magnésium | 0,5 % |
| Tréhalose | 1 % |
| BY 29-122 (à 63 % de matière active) | 5 % |
| Conservateurs | 0,4 % |
| Eau | qsp 100 % |

Mode opératoire : on chauffe séparément à 75°C les phases huileuse et aqueuse, puis on émulsionne en dispersant sous agitation vigoureuse la phase aqueuse dans la phase huileuse.

On obtient ainsi une crème onctueuse, douce et riche à l'application. Elle pénètre facilement en apportant immédiatement un effet nutritif apaisant. Elle est particulièrement appropriée pour le soin des peaux sèches et sensibles.

## Revendications

1. Composition contenant dans un milieu physiologiquement acceptable, au moins une phase aqueuse, au moins un sucre et/ou un dérivé de sucre et une suspension aqueuse de particules d'organopolysiloxane élastomère au moins partiellement réticulé, ladite suspension étant présente dans la phase aqueuse de la composition.

2. Composition selon la revendication 1, **caractérisée en ce que** l'organopolysiloxane élastomère est obtenu par réaction d'addition et de réticulation, en présence d'un catalyseur, d'au moins :
- un organopolysiloxane (i) ayant deux groupements vinyliques en position α-ω de la chaîne siliconée par molécule ; et
- un organosiloxane (ii) ayant au moins un atome d'hydrogène lié à un atome de silicium par molécule.

3. Composition selon la revendication précédente, **caractérisée en ce que** l'organopolysiloxane (i) est choisi parmi les polydiméthylsiloxanes.

4. Composition selon la revendication 2 ou 3, **caractérisée en ce que** l'organopolysiloxane (i) est un α-ω-diméthylvinyl polydiméthylsiloxane.

5. Composition selon l'une quelconque des revendications 2 à 4, **caractérisée en ce que** la suspension aqueuse de particules d'organopolysiloxane est obtenue selon les étapes suivantes :
- (a) mélange de l'organopolysiloxane (i) et de l'organosiloxane (ii) ;
- (b) ajout de la phase aqueuse contenant un émulsifiant au mélange de l'étape (a);
- (c) émulsification de la phase aqueuse et dudit mélange ;
- (d) ajout d'eau chaude à l'émulsion de l'étape (c) ; et
- (e) polymérisation de l'organopolysiloxane (i) et de l'organosiloxane (ii) en émulsion en présence d'un catalyseur de platine.

6. Composition selon la revendication précédente, **caractérisée en ce que** l'étape (c) est obtenue en présence d'un émulsifiant non-ionique.

7. Composition selon l'une quelconque des revendications précédentes, **caractérisée en ce que** les particules d'organosiloxane ont une taille allant de 0,1 à 500 µm.

8. Composition selon l'une quelconque des revendications précédentes, **caractérisée en ce que** les particules d'organopolysiloxane présentent une dureté inférieure ou égale à 80 mesurable selon la description p. 3.

9. Composition selon l'une quelconque des revendications précédentes, **caractérisée en ce que** les particules d'organosiloxane sont présentes en une quantité en matière active allant de 0,1 à 30 % en poids par rapport au poids total de la composition.

10. Composition selon l'une quelconque des revendications précédentes, **caractérisée en ce que** le sucre est choisi parmi le sucrose, le glucose, le galactose, le ribose, le fucose, le tréhalose, le maltose, le fructose, le mannose, l'arabinose, le xylose, le lactose, leurs dérivés alkylés, les composés les contenant, et leurs mélanges.

11. Composition selon l'une quelconque des revendications précédentes, **caractérisée en ce que** le dérivé de sucre est choisi parmi les esters gras de sucre éventuellement oxyalkylénés ou polyglycérolés, les éthers gras de sucre ou leurs mélanges.

12. Composition selon l'une quelconque des revendications précédentes, **caractérisée en ce que** le dérivé de sucre est choisi parmi les esters d'acide gras en C₁₂ à C₂₂ linéaires ou ramifiés, saturés ou insaturés et de sucrose, de maltose, de glucose, de fructose, de mannose, de galactose, d'arabinose, de xylose, de lactose, de tréhalose, ou de méthylglucose, les éthers d'alcool gras en C₈-C₂₂ et de glucose, de maltose, de sucrose ou de fructose et les éthers d'alcool gras en C₁₄-C₂₂ et de méthylglucose, et leurs mélanges.

13. Composition selon l'une quelconque des revendications précédentes, **caractérisée par le fait que** la quantité de sucre et/ou dérivé de sucre va de 0,1 à 20 % en poids par rapport au poids total de la composition.

14. Composition selon l'une quelconque des revendications précédentes, **caractérisée par le fait qu'**elle se présente sous forme d'une émulsion H/E ou E/H.

15. Composition selon la revendication précédente, **caractérisée en ce que** la phase huileuse représente de 1 à 50 % du poids total de la composition.

16. Composition selon l'une quelconque des revendications précédentes, **caractérisée en ce qu'**elle constitue une composition cosmétique.

17. Utilisation cosmétique de la composition selon l'une quelconque des revendications 1 à 16, pour le traitement, la protection, le soin, le démaquillage et/ou le nettoyage de la peau, des lèvres et/ou des cheveux.

18. Procédé de traitement cosmétique de la peau, des cheveux, et/ou des lèvres, **caractérisé par le fait qu'**on applique sur la peau, les cheveux et/ou les lèvres, une composition selon l'une quelconque des revendications 1 à 16.

19. Utilisation de la composition selon l'une quelconque des revendications 1 à 16, pour la fabrication d'une composition destinée au soin des peaux sèches et/ou des lèvres sèches et/ou des peaux sensibles.

20. Utilisation cosmétique d'une suspension aqueuse de particules d'organopolysiloxane solide élastomère au moins partiellement réticulé, dans une composition contenant au moins un sucre et/ou un dérivé de sucre, afin d'éliminer le toucher collant dû à ce sucre et/ou dérivé de sucre.

## Claims

1. Composition comprising, in a physiologically acceptable medium, at least one aqueous phase, at least one sugar and/or one sugar derivative and an aqueous suspension of particles of at least partially crosslinked organopolysiloxane elastomer, the said suspension being present in the aqueous phase of the composition.

2. Composition according to Claim 1, **characterized in that** the organopolysiloxane elastomer is obtained by an addition and crosslinking reaction, in the presence of a catalyst, of at least:
- one organopolysiloxane (i) having two vinyl groups in the α,ω-position of the silicone chain per molecule; and
- one organosiloxane (ii) having at least one hydrogen atom bonded to a silicon atom per molecule.

3. Composition according to the preceding claim, **characterized in that** the organopolysiloxane (i) is chosen from polydimethylsiloxanes.

4. Composition according to Claim 2 or 3, **characterized in that** the organopolysiloxane (i) is an α,ω-dimethylvinylpolydimethylsiloxane.

5. Composition according to any one of Claims 2 to 4, **characterized in that** the aqueous suspension of organopolysiloxane particles is obtained according to the following stages:
- (a) mixing the organopolysiloxane (i) and the organosiloxane (ii);
- (b) adding the aqueous phase comprising an emulsifier to the mixture from stage (a);
- (c) emulsifying the aqueous phase and the said mixture;
- (d) adding warm water to the emulsion from stage (c) ; and
- (e) polymerizing the organopolysiloxane (i) and the organosiloxane (ii) in an emulsion in the presence of a platinum catalyst.

6. Composition according to the preceding claim, **characterized in that** stage (c) is carried out in the presence of a nonionic emulsifier.

7. Composition according to any one of the preceding claims, **characterized in that** the organosiloxane particles have a size ranging from 0.1 to 500 µm.

8. Composition according to any one of the preceding claims, **characterized in that** the organopolysiloxane particles exhibit a hardness of less than or equal to 80, measurable according to p.3 of the description.

9. Composition according to any one of the preceding claims, **characterized in that** the organosiloxane particles are present in an amount of active material ranging from 0.1 to 30% by weight with respect to the total weight of the composition.

10. Composition according to any one of the preceding claims, **characterized in that** the sugar is chosen from sucrose, glucose, galactose, ribose, fucose, trehalose, maltose, fructose, mannose, arabinose, xylose, lactose, their alkylated derivatives, the compounds comprising them and their mixtures.

11. Composition according to any one of the preceding claims, **characterized in that** the sugar derivative is chosen from sugar fatty esters, which are optionally oxyalkylenated or polyglycerolated, sugar fatty ethers or their mixtures.

12. Composition according to any one of the preceding claims, **characterized in that** the sugar derivative is chosen from esters of linear or branched and saturated or unsaturated C₁₂ to C₂₂ fatty acids and of sucrose, maltose, glucose, fructose, mannose, galactose, arabinose, xylose, lactose, trehalose or methylglucose, ethers of C₈-C₂₂ fatty alcohol and of glucose, maltose, sucrose or fructose and ethers of C₁₄-C₂₂ fatty alcohol and of methylglucose, and their mixtures.

13. Composition according to any one of the preceding claims, **characterized in that** the amount of sugar and/or sugar derivative ranges from 0.1 to 20% by weight with respect to the total weight of the composition.

14. Composition according to any one of the preceding claims, **characterized in that** it is provided in the form of an O/W or W/O emulsion.

15. Composition according to the preceding claim, **characterized in that** the oily phase represents from 1 to 50% of the total weight of the composition.

16. Composition according to any one of the preceding claims, **characterized in that** it constitutes a cosmetic composition.

17. Cosmetic use of the composition according to any one of Claims 1 to 16 for treating, protecting, caring for, removing make-up from and/or cleaning the skin, lips and/or hair.

18. Process for the cosmetic treatment of the skin, hair and/or lips, **characterized in that** a composition according to any one of Claims 1 to 16 is applied to the skin, hair and/or lips.

19. Use of the composition according to any one of Claims 1 to 16 in the manufacture of a composition intended for caring for dry skin and/or dry lips and/or sensitive skin.

20. Cosmetic use of an aqueous suspension of particles of at least partially crosslinked solid organopolysiloxane elastomer, in a composition comprising at least one sugar and/or one sugar derivative, in order to eliminate the sticky feel due to this sugar and/or sugar derivative.

## Patentansprüche

1. Zusammensetzung, die in einem physiologisch akzeptablen Medium mindestens eine wässerige Phase, mindestens einen Zucker und/oder ein Zuckerderivat und eine wässerige Suspension von Partikeln eines zumindest teilweise vernetzten elastomeren Polyorganosiloxans enthält, wobei die Suspension in der wässerigen Phase der Zusammensetzung vorliegt.

2. Zusammensetzung nach Anspruch 1, **dadurch gekennzeichnet, dass** das elastomere Polyorganosiloxan durch Addition und Vernetzung mindestens.
- eines Polyorganosiloxans (i), das pro Molekül zwei Vinylgruppen in α-ω-Stellung der Siliconkette aufweist, und
- eines Polyorganosiloxans (ii), das pro Molekül mindestens ein Wasserstoffatom aufweist, das an ein Siliciumatom gebunden ist, hergestellt wird, wobei die Umsetzung in Gegenwart eines Katalysators erfolgt.

3. Zusammensetzung nach dem vorhergehenden Anspruch, **dadurch gekennzeichnet, dass** das Polyorganosiloxan (i) unter Polydimethylsiloxanen ausgewählt ist.

4. Zusammensetzung nach Anspruch 2 oder 3, **dadurch gekennzeichnet, dass** das Polyorganosiloxan (i) ein α-ω-Dimethylvinylpolydimethylsiloxan ist.

5. Zusammensetzung nach einem der Ansprüche 2 bis 4, **dadurch gekennzeichnet, dass** die wässerige Suspension von Polyorganosiloxan-Partikeln in den folgenden Schritten hergestellt wird:
- (a) Mischen des Polyorganosiloxans (i) mit dem Polyorganosiloxan (ii),
- (b) Zugabe der wässerigen Phase, die einen Emulgator enthält, zu dem Gemisch von Schritt (a),
- (c) Emulgieren der wässerigen Phase und des Gemischs,
- (d) Zugabe von warmem Wasser zu der Emulsion von Schritt (c) und
- (e) Polymerisation des in Emulsion befindlichen Polyorganosiloxans (i) und Polyorganosiloxans (ii) in Gegenwart eines Platinkatalysators.

6. Zusammensetzung nach dem vorhergehenden Anspruch, **dadurch gekennzeichnet, dass** der Schritt (c) in Gegenwart eines nichtionischen Emulgators durchgeführt wird.

7. Zusammensetzung nach einem der vorhergehenden Ansprüche, **dadurch gekennzeichnet, dass** die Polyorganosiloxan-Partikel eine Größe von 0,1 bis 500 µm aufweisen.

8. Zusammensetzung nach einem der vorhergehenden Ansprüche, **dadurch gekennzeichnet, dass** die Polyorganosiloxan-Partikel eine nach der Beschreibung auf Seite 5 bestimmbare Härte von kleiner oder gleich 80 aufweisen.

9. Zusammensetzung nach einem der vorhergehenden Ansprüche, **dadurch gekennzeichnet, dass** die Polyorganosiloxan-Partikel in einem als Wirkstoff ausgedrückten Mengenanteil von 0,1 bis 30 Gew.-%, bezogen auf das Gesamtgewicht der Zusammensetzung, vorliegen.

10. Zusammensetzung nach einem der vorhergehenden Ansprüche, **dadurch gekennzeichnet, dass** der Zucker unter Saccharose, Glucose, Galactose, Ribose, Fucose, Trehalose, Maltose, Fructose, Mannose, Arabinose, Xylose, Lactose und deren alkylierten Derivaten, Verbindungen, die sie enthalten, und deren Gemischen ausgewählt ist.

11. Zusammensetzung nach einem der vorhergehenden Ansprüche, **dadurch gekennzeichnet, dass** das Zuckerderivat unter gegebenenfalls alkoxylierten oder mehrfach mit Glycerin veretherten Fettestern eines Zuckers, Fettethern eines Zückers oder den Gemischen dieser Verbindungen ausgewählt ist.

12. Zusammensetzung nach einem der vorhergehenden Ansprüche, **dadurch gekennzeichnet, dass** das Zuckerderivat unter geradkettigen oder verzweigten, gesättigten oder ungesättigten Estern von Fettsäuren mit 12 bis 22 Kohlenstoffatomen mit Saccharose, Maltose, Glucose, Fructose, Mahnose, Galactose, Arabinose, Xylose, Lactose, Trehalose oder Methylglucose, Ethern von Fettalkoholen mit 8 bis 22 Kohlenstoffatomen und Glucose, Maltose, Saccharose oder Fructose und Ethern von Fettalkoholen mit 14 bis 22 Kohlenstoffatomen und Methylglucose sowie deren Gemischen ausgewählt ist.

13. Zusammensetzung nach einem der vorhergehenden Ansprüche, **dadurch gekennzeichnet, dass** der Mengenanteil des Zuckers und/oder des Zuckerderivats im Bereich von 0,1 bis 20 Gew.-%, bezogen auf das Gesamtgewicht der Zusammensetzung, liegt.

14. Zusammensetzung nach einem der vorhergehenden Ansprüche, **dadurch gekennzeichnet, dass** sie in Form einer O/W-Emulsion oder einer W/O-Emulsion vorliegt.

15. Zusammensetzung nach dem vorhergehenden Anspruch, **dadurch gekennzeichnet, dass** die Ölphase 1 bis 50 % des Gesamtgewichts der Zusammensetzung ausmacht.

16. Zusammensetzung nach einem der vorhergehenden Ansprüche, **da-durch gekennzeichnet, dass** sie eine kosmetische Zusammensetzung darstellt.

17. Kosmetische Verwendung der Zusammensetzung nach einem der Ansprüche 1 bis 16 zur Behandlung, zum Schutz, zur Pflege, zum Abschminken und/oder zur Reinigung der Haut, der Lippen und/ oder der Haare.

18. Verfahren zur kosmetischen Behandlung der Haut, der Haare und/oder der Lippen, **dadurch gekennzeichnet, dass** eine Zusammensetzung nach einem der Ansprüche 1 bis 16 auf die Haut, die Haare und/oder die Lippen aufgebracht wird.

19. Verwendung der Zusammensetzung nach einem der Ansprüche 1 bis 16 zur Herstellung einer Zusammensetzung, die für die Pflege trockener Haut und/oder trockener Lippen und/oder empfindlicher Haut bestimmt ist.

20. Kosmetische Verwendung einer wässerigen Suspension von Partikeln eines zumindest teilweise vernetzten festen elastomeren Polyorganosiloxans in einer Zusammensetzung, die mindestens einen Zucker und/oder ein Zuckerderivat enthält, um den klebrigen Eindruck zu beseitigen, der von dem Zucker und/oder dem Zuckerderivat herrührt.
